# EUROPEAN PATENT APPLICATION

(11) **EP 4 385 413 A1**
(43) Date of publication of application: **19.06.2024**
(21) Application number: 22873168.3
(22) Date of filing: 21.09.2022
(51) Int. Cl.: A61B 5/346, A61B 5/02, A61B 5/00, G16H 50/20, G16H 50/50, G06N 3/08, G06N 3/04

(54) **METHOD, COMPUTER PROGRAM, AND DEVICE FOR CONTINUOUSLY MEASURING BODY CONDITION ON BASIS OF DEEP LEARNING**

(30) Priority: 25.09.2021 KR 20210126782; 20.09.2022 KR 20220118514
(71) Applicant: Medical AI Co., Ltd., Seoul 06302 (KR)
(72) Inventor: KWON, Joonmyoung, 163 SEOUL (KR)
(74) Representative: Isarpatent
(86) International application number: PCT/KR2022/014109
(87) International publication number: WO 2023/048463

(57) **Abstract**

According to an embodiment of the present disclosure, there are disclosed a method, computer program and device for measuring a continuous body condition based on deep learning, which are performed by a computing device. The method includes: acquiring electrocardiogram data; and inferring a body condition corresponding to the occurrence or progress of a disease in a subject, whose electrocardiogram data was measured, based on the electrocardiogram data by using a pre-trained neural network model. The neural network model has been trained based on at least one of a first feature related to biological information representing a body characteristic having a correlation with the disease and a second feature related to pathological information reflecting the degree of progress of the disease therein.

## Description

### Technical Field

The present disclosure relates to deep learning technology in the medical field, and more particularly to a method, computer program, and device for measuring a continuous body condition based on deep learning to represent a body characteristic for a disease in the form of a continuous numerical value.

### Background Art

Electrocardiogram signals are the electrical activities of the heart that occur during a heartbeat cycle and are plotted on a graph. Accordingly, we can observe the structural and functional aspects of the heart through electrocardiogram signals. Therefore, electrocardiogram signals are used to diagnose various diseases, including heart diseases such as arrhythmia and myocardial infarction.

General physical disease, including heart disease, refers to a situation in which a continuously changing body condition worsens by a set reference value for being diagnosed as a disease. In other words, a body condition changes continuously in an analog-like manner rather than a digital manner based on 0 or 1, whereas a disease is determined based on an artificially set reference value. For example, in the case of heart disease, the condition of the coronary arteries that supply blood to the heart changes continuously, and diseases such as arteriosclerosis, angina pectoris, and myocardial infarction are determined depending on the degree of stenosis by which the coronary arteries are narrowed.

Various technologies are being developed based on deep learning to predict physical disease. However, most of the prior technologies are each intended to determine the presence/absence of a disease determined according to an artificially set reference. In other words, the presence/absence of a disease is determined by classifying a continuously changing body condition based on a specific reference. A deep learning model trained using this principle can only predict the presence/absence of a disease but cannot predict an overall body condition for the disease.

The purpose of the prediction of disease is to prevent disease by estimating the possibility that disease will occur. Accordingly, in the field of the prediction of disease, accurately predicting a body condition related to a disease is as important as predicting the presence/absence of the disease. In other words, we need to predict a continuously changing body condition related to disease.

### Disclosure

### Technical Problem

The present disclosure was conceived in response to the above-mentioned background technology, and an object of the present disclosure is to provide a method that individually predicts biological information about a body characteristic and pathological information about a disease based on electrocardiogram data, and combines them, thereby measuring a body condition as a continuous numerical value.

However, the objects to be accomplished by the present disclosure are not limited to the object mentioned above, and other objects not mentioned may be clearly understood based on the following description.

### Technical Solution

According to an embodiment of the present disclosure for accomplishing the above-described object, there is disclosed a method of measuring a continuous body condition based on deep learning, which is performed by a computing device. The method includes: acquiring electrocardiogram data; and inferring a body condition corresponding to the occurrence or progress of a disease in a subject, whose electrocardiogram data was measured, based on the electrocardiogram data by using a pre-trained neural network model. The neural network model has been trained based on at least one of a first feature related to biological information representing a body characteristic having a correlation with the disease and a second feature related to pathological information reflecting the degree of progress of the disease therein.

Alternatively, the neural network model may include at least one first sub-model trained to output the first feature based on the electrocardiogram data; and the at least one first sub-model may be configured in accordance with the number of factors to individually output numerical values for one or more factors included in the biological information.

Alternatively, the neural network model may further include at least one second sub-model trained to output the second feature based on the electrocardiogram data; and the at least one second sub-model may be configured in accordance with the number of factors to individually output numerical values for one or more factors included in the pathological information.

Alternatively, the neural network model may further include a third sub-model trained to represent a body condition continuously changing according to the occurrence or progress of the disease in the form of a numerical value based on the first feature, which is the output of the first sub-model, and the second feature, which is the output of the second sub-model.

Alternatively, the third sub-model may receive a third feature generated by combining the first feature and the second feature based on weights determined according to the type of disease, and may output the numerical value.

Alternatively, each of the first and second sub-models may be trained based on self-supervised learning that is performed using training data including unlabeled samples.

Alternatively, the disease may include a cardiovascular disease.

Alternatively, the biological information may include at least one of age, gender, height, and weight as a body characteristic factor related to a coronary artery disease included in the cardiovascular disease.

Alternatively, the pathological information may include at least one of the presence/absence of myocardial infarction, the degree of vascular calcification, the stability of blood clots, the intravascular velocity of coronary arteries, and the degree of stenosis of coronary arteries as a pathological characteristic factor that reflects the degree of progress of a coronary artery disease included in the cardiovascular disease.

According to an embodiment of the present disclosure for accomplishing the above-described object, there is disclosed a computer program that is stored in a computer-readable storage medium. The computer program performs the operations of measuring a continuous body condition based on deep learning when executed on one or more processors. In this case, the operations include: the operations of: acquiring electrocardiogram data; and inferring a body condition corresponding to an occurrence or progress of a disease in a subject, whose electrocardiogram data was measured, based on the electrocardiogram data by using a pre-trained neural network model. The neural network model has been trained based on at least one of a first feature related to biological information representing a body characteristic having a correlation with the disease and a second feature related to pathological information reflecting the degree of progress of the disease therein.

According to an embodiment of the present disclosure for accomplishing the above-described object, there is disclosed a computing device for measuring a continuous body condition based on deep learning. The computing device includes: a processor including at least one core; memory including program codes that are executable on the processor; and a network unit configured to acquire electrocardiogram data. The processor infers a body condition corresponding to the occurrence or progress of a disease in a subject, whose electrocardiogram data was measured, based on the electrocardiogram data by using a neural network model trained based on at least one of a first feature related to biological information representing a body characteristic having a correlation with the disease and a second feature related to pathological information reflecting the degree of progress of the disease therein.

### Advantageous Effects

The present disclosure may provide a method of preparing for the previous or subsequent stage of disease by individually inferring body factors and pathological factors for disease using electrocardiogram data and thus identifying factors affecting a body condition in an explanatory manner.

In addition, the present disclosure may provide a method of preparing for the previous or subsequent stage of disease by representing a patient's body condition related to disease to be predicted in the form of a continuous numerical value.

### Description of Drawings

FIG. 1 is a block diagram of a computing device according to an embodiment of the present disclosure;
FIG. 2 is a block diagram showing a neural network model according to an embodiment of the present disclosure;
FIG. 3 is a block diagram showing the internal configuration of a neural network model according to an embodiment of the present disclosure;
FIG. 4 is a flowchart showing an inference method for a neural network model according to an embodiment of the present disclosure; and
FIG. 5 is a flowchart showing an inference method for sub-models constituting a neural network model according to an embodiment of the present disclosure.

### Mode for Invention

Embodiments of the present disclosure will be described in detail below with reference to the accompanying drawings so that those having ordinary skill in the art of the present disclosure (hereinafter referred to as those skilled in the art) can easily implement the present disclosure. The embodiments presented in the present disclosure are provided to enable those skilled in the art to use or practice the content of the present disclosure. Accordingly, various modifications to embodiments of the present disclosure will be apparent to those skilled in the art. That is, the present disclosure may be implemented in various different forms and is not limited to the following embodiments.

The same or similar reference numerals denote the same or similar components throughout the specification of the present disclosure. Additionally, in order to clearly describe the present disclosure, reference numerals for parts that are not related to the description of the present disclosure may be omitted in the drawings.

The term "or" used herein is intended not to mean an exclusive "or" but to mean an inclusive "or." That is, unless otherwise specified herein or the meaning is not clear from the context, the clause "X uses A or B" should be understood to mean one of the natural inclusive substitutions. For example, unless otherwise specified herein or the meaning is not clear from the context, the clause "X uses A or B" may be interpreted as any one of a case where X uses A, a case where X uses B, and a case where X uses both A and B.

The term "and/or" used herein should be understood to refer to and include all possible combinations of one or more of listed related concepts.

The terms "include" and/or "including" used herein should be understood to mean that specific features and/or components are present. However, the terms "include" and/or "including" should be understood as not excluding the presence or addition of one or more other features, one or more other components, and/or combinations thereof.

Unless otherwise specified herein or unless the context clearly indicates a singular form, the singular form should generally be construed to include "one or more."

The term "N-th (N is a natural number)" used herein can be understood as an expression used to distinguish the components of the present disclosure according to a predetermined criterion such as a functional perspective, a structural perspective, or the convenience of description. For example, in the present disclosure, components performing different functional roles may be distinguished as a first component or a second component. However, components that are substantially the same within the technical spirit of the present disclosure but should be distinguished for the convenience of description may also be distinguished as a first component or a second component.

The term "acquisition" used herein can be understood to mean not only receiving data over a wired/wireless communication network connecting with an external device or a system, but also generating data in an on-device form.

Meanwhile, the term "module" or "unit" used herein may be understood as a term referring to an independent functional unit processing computing resources, such as a computer-related entity, firmware, software or part thereof, hardware or part thereof, or a combination of software and hardware. In this case, the "module" or "unit" may be a unit composed of a single component, or may be a unit expressed as a combination or set of multiple components. For example, in the narrow sense, the term "module" or "unit" may refer to a hardware component or set of components of a computing device, an application program performing a specific function of software, a procedure implemented through the execution of software, a set of instructions for the execution of a program, or the like. Additionally, in the broad sense, the term "module" or "unit" may refer to a computing device itself constituting part of a system, an application running on the computing device, or the like. However, the above-described concepts are only examples, and the concept of "module" or "unit" may be defined in various manners within a range understandable to those skilled in the art based on the content of the present disclosure.

The term "model" used herein may be understood as a system implemented using mathematical concepts and language to solve a specific problem, a set of software units intended to solve a specific problem, or an abstract model for a process intended to solve a specific problem. For example, a neural network "model" may refer to an overall system implemented as a neural network that is provided with problem-solving capabilities through training. In this case, the neural network may be provided with problem-solving capabilities by optimizing parameters connecting nodes or neurons through training. The neural network "model" may include a single neural network, or a neural network set in which multiple neural networks are combined together.

The foregoing descriptions of the terms are intended to help to understand the present disclosure. Accordingly, it should be noted that unless the above-described terms are explicitly described as limiting the content of the present disclosure, the terms in the content of the present disclosure are not used in the sense of limiting the technical spirit of the present disclosure.

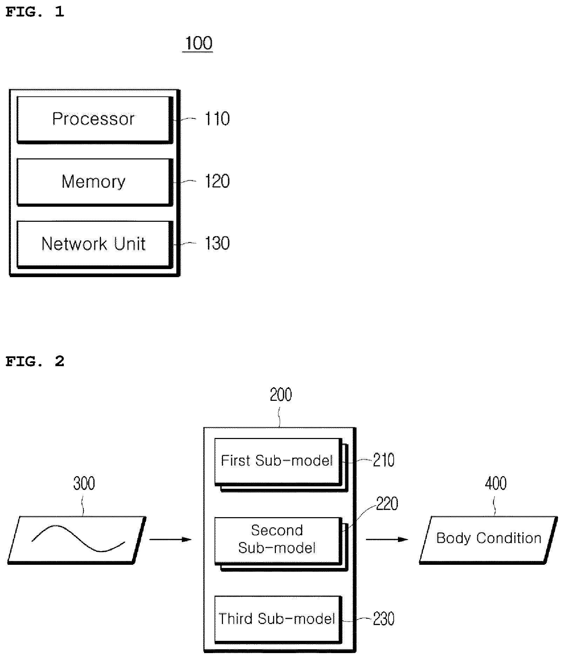

FIG. 1 is a block diagram of a computing device according to an embodiment of the present disclosure.

A computing device 100 according to an embodiment of the present disclosure may be a hardware device or part of a hardware device that performs the comprehensive processing and calculation of data, or may be a software-based computing environment that is connected to a communication network. For example, the computing device 100 may be a server that performs an intensive data processing function and shares resources, or may be a client that shares resources through interaction with a server. Furthermore, the computing device 100 may be a cloud system in which a plurality of servers and clients interact with each other and comprehensively process data. Since the above descriptions are only examples related to the type of computing device 100, the type of computing device 100 may be configured in various manners within a range understandable to those skilled in the art based on the content of the present disclosure.

Referring to FIG. 1, the computing device 100 according to an embodiment of the present disclosure may include a processor 110, memory 120, and a network unit 130. However, FIG. 1 shows only an example, and the computing device 100 may include other components for implementing a computing environment. Furthermore, only some of the components disclosed above may be included in the computing device 100.

The processor 110 according to an embodiment of the present disclosure may be understood as a configuration unit including hardware and/or software for performing computing operation. For example, the processor 110 may read a computer program and perform data processing for machine learning. The processor 110 may process computational processes such as the processing of input data for machine learning, the extraction of features for machine learning, and the calculation of errors based on backpropagation. The processor 110 for performing such data processing may include a central processing unit (CPU), a general purpose graphics processing unit (GPGPU), a tensor processing unit (TPU), an application specific integrated circuit (ASIC), or a field programmable gate array (FPGA). Since the types of processor 110 described above are only examples, the type of processor 110 may be configured in various manners within a range understandable to those skilled in the art based on the content of the present disclosure.

The processor 110 may measure the continuous body condition of a subject, whose electrocardiogram data was measured, using a neural network model based on the electrocardiogram data. The processor 110 may receive electrocardiogram data as input and train the neural network model to estimate a body condition related to disease, for example, whether the disease has occurred or a body condition corresponding to the progress of the disease. In this case, whether the disease has occurred may be a discrete value, and the progress of the disease may be a continuous value that changes over time. The processor 110 may represent a disease or infer a continuous body condition affecting the disease using the neural network model.

According to the present disclosure, the processor 110 not only predicts the occurrence of a disease expressed as a discrete value, but also predicts a body condition according to a continuously progressing disease, so that the possibility that a disease develops can be accurately predicted. Accordingly, through the processor 110 of the present disclosure, we can establish disease prevention measures and treatment plans in detail.

The processor 110 may individually train a plurality of sub-models to predict a body condition. For example, the processor 110 may train a first sub-model configured to output a first feature related to biological information, representing the body characteristics of a subject for the measurement of electrocardiogram data, by using electrocardiogram data. Furthermore, the processor 110 may train a second sub-model configured to output a second feature related to pathological information affecting the body changes caused by a disease, that is, the progress of the disease, by using electrocardiogram data. Moreover, the processor 110 may train a third sub-model configured to infer a body condition related to a disease by using the first feature output from the first sub-model and the second feature output from the second sub-model.

The first characteristic may include at least one factor included in biological information and a numerical value corresponding to the factor. Biological factors may vary depending on the disease. For example, biological factors may include age, height, weight, etc. The second feature may include at least one factor included in pathological information and a numerical value corresponding to the factor. Pathological factors may vary depending on the disease. For example, in the case of cardiovascular disease, pathological factors may include the presence/absence of myocardial infarction, the degree of vascular calcification, the stability of blood clots, the intravascular velocity of coronary arteries, the degree of stenosis of coronary arteries, etc.

The processor 110 may perform training based on supervised learning by inputting training data, including samples and labels corresponding to the samples, to the first sub-model and the second sub-model. In this case, the sample input to the first sub-model may be electrocardiogram data, and the label may be the first feature. The sample input to the second sub-model may be electrocardiogram data, and the label may be the second feature. Furthermore, the processor 110 may perform training based on self-supervised learning by inputting training data, including unlabeled samples, to the first sub-model and the second sub-model. In this case, the first sub-model may extract the first feature from the electrocardiogram data, and the second sub-model may extract the second feature from the electrocardiogram data. Factors extracted by the first sub-model and the second sub-model may vary depending on the type of disease.

The neural network model, the first sub-model, the second sub-model, and the third sub-model may each include at least one neural network. The neural network may include at least one of neural networks such as a Deep Neural Network (DNN), a Recurrent Neural Network (RNN), a Bidirectional Recurrent Deep Neural Network (BRDNN), a Multilayer Perceptron (MLP), a Convolutional Neural Network (CNN), a transformer, etc., but is not limited thereto.

According to the present disclosure, the processor 110 may individually train and infer neural network models corresponding to factors that may act as risk factors for disease. Accordingly, we may accurately determine the degree, to which each factor affects the disease, through the processor 110. Additionally, the processor 110 may provide basic data for the prevention and treatment of disease by accurately targeting factors that affect the disease.

The memory 120 according to an embodiment of the present disclosure may be understood as a configuration unit including hardware and/or software for storing and managing data that is processed in the computing device 100. That is, the memory 120 may store any type of data generated or determined by the processor 110 and any type of data received by the network unit 130. For example, the memory 120 may include at least one type of storage medium of a flash memory type, hard disk type, multimedia card micro type, and card type memory, random access memory (RAM), static random access memory (SRAM), read-only memory (ROM), electrically erasable programmable read-only memory (EEPROM), programmable read-only memory (PROM), magnetic memory, a magnetic disk, and an optical disk. Furthermore, the memory 120 may include a database system that controls and manages data in a predetermined system. Since the types of memory 120 described above are only examples, the type of memory 120 may be configured in various manners within a range understandable to those skilled in the art based on the content of the present disclosure.

The memory 120 may structure, organize, and manage data necessary for the processor 110 to perform computation, the combination of data, and program codes executable on the processor 110. For example, the memory 120 may store medical data received through the network unit 130, which will be described later. The memory 120 may store program codes configured to operate the neural network model to receive medical data and perform learning, program codes configured to operate the neural network model to receive medical data and perform inference in accordance with the purpose of use of the computing device 100, processed data generated as program codes are executed, etc.

The network unit 130 according to an embodiment of the present disclosure may be understood as a configuration unit that transmits and receives data through any type of known wired/wireless communication system. For example, the network unit 130 may perform data transmission and reception using a wired/wireless communication system such as a local area network (LAN), a wideband code division multiple access (WCDMA) network, a long term evolution (LTE) network, the wireless broadband Internet (WiBro), a 5th generation mobile communication (5G) network, a ultra wide-band wireless communication network, a ZigBee network, a radio frequency (RF) communication network, a wireless LAN, a wireless fidelity network, a near field communication (NFC) network, or a Bluetooth network. Since the above-described communication systems are only examples, the wired/wireless communication system for the data transmission and reception of the network unit 130 may be applied in various manners other than the above-described examples.

The network unit 130 may receive data necessary for the processor 110 to perform computation through wired/wireless communication with any system or client or the like. Furthermore, the network unit 130 may transmit data generated through the computation of the processor 110 through wired/wireless communication with any system or client or the like. For example, the network unit 130 may receive medical data through communication with a database within a hospital environment, a cloud server that performs tasks such as the standardization of medical data, or a computing device. The network unit 130 may transmit the output data of the neural network model, intermediate data and processed data acquired from the computation process of the processor 110, etc. through communication with the above-described database, server, or computing device.

FIG. 2 is a block diagram showing a neural network model according to an embodiment of the present disclosure.

Referring to FIG. 2, the neural network model 200 may receive electrocardiogram data 300, and may output a body condition 400 related to the disease of a subject, whose electrocardiogram data 300 was measured, as a continuous numerical value.

In the present specification, electrocardiogram (ECG) data may include electrocardiogram signals that enable the presence/absence of a disease to be determined by checking for abnormalities in a conduction system extending from the heart to the electrodes through the measurement of electrical signals generated in the heart. The electrocardiogram data 300 may be acquired from an electrocardiogram measurement device, or may be acquired over a network.

The neural network model 200 may include a first sub-model 210 that outputs a first feature regarding biological information related to a disease, a second sub-model 220 that outputs a second feature regarding pathological information related to a disease, and a third sub-model 230 that outputs a continuously changing body condition 400 as a numerical value for the diagnosis of a disease by using the output value of the first sub-model 210 and the output value of the second sub-model 220.

Each of the first sub-model 210 and the second sub-model 220 may be configured to include a plurality of sub-models depending on the information to be output. For example, the first sub-model 210 may output a first feature corresponding to each of the body characteristic factors representing biological characteristics related to a disease from the electrocardiogram data 300. In this case, the number of first sub-models 210 may correspond to the number of body characteristic factors. The plurality of first sub-models 210 may output numerical values for body characteristic factors, respectively. For example, the second sub-model 220 may output a second feature corresponding to each of the pathological characteristic factors determined according to the degree of progress of a disease from the electrocardiogram data 300. In this case, the number of second sub-models 220 may correspond to the number of pathological characteristic factors. The plurality of second sub-models 220 may output numerical values for pathological characteristic factors, respectively.

Meanwhile, the number of risk factors related to a disease may vary depending on the type of disease. Accordingly, the first sub-model and the second sub-model may be configured in accordance with the number of factors through the neural network model according to the present disclosure. We may modularize and manage the first sub-model and the second sub-model.

The third sub-model 230 may output the body condition 400 related to the disease as a numerical value using the first feature, which is the output of the first sub-model 210, and the second feature, which is the output of the second sub-model 220. The third sub model 230 may receive the third feature, generated by combining the first feature and the second feature, as input and output the body condition 400. In this case, the first feature and the second feature may be combined into a third feature with weights assigned thereto according to the type of disease. In other words, the processor 110 may adjust the weights for the combination of the first feature and the second feature depending on the type of disease. The third sub-model 230 may predict the body condition 400 that continuously changes in accordance with the type of disease based on the first and second features whose proportions have been adjusted according to the type of disease. The value output from the third sub model 230 may be a continuous numerical value indicating the body condition 400 for the disease. Accordingly, medical staff may determine whether a disease has occurred or the progress of the disease based on the corresponding numerical value.

That is, the neural network model 200 according to the present disclosure does not simply predict whether a disease has occurred according to artificially set criteria, but may predict a body condition by comprehensively identifying factors related to the disease. Using the neural network model 200, we may accurately identify complex changes in the body condition that occur as various factors related to the disease affect the body.

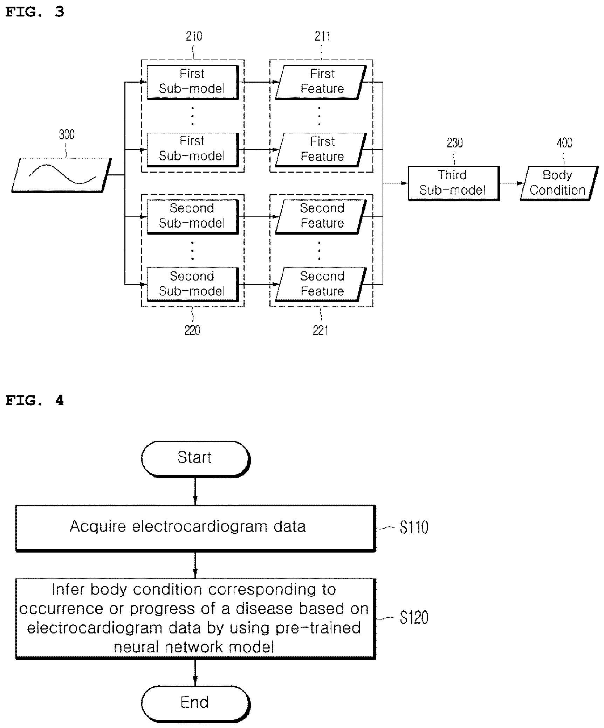

FIG. 3 is a block diagram showing the internal configuration of a neural network model according to an embodiment of the present disclosure.

Referring to FIG. 3, the neural network model 200 may include a plurality of first sub-models 210 trained to output first features 211, and a plurality of second sub-models 220 trained to output second features 221, and a third sub-model 230 trained to output the body condition 400 by combining the first features 211 and the second features 221.

The first features 211 may include factors included in biological information and numerical values corresponding to the factors. The second features 221 may include factors included in pathological information and numerical values corresponding to the factors.

The types of body characteristic factors on which the first sub-models 210 are individually trained may be determined by labels included in training data, or may be directly extracted by the first sub-models 210. In the same manner, the types of pathological characteristic factors on which the second sub-models 220 are individually trained may be determined by labels included in training data, or may be directly extracted by the second sub-models 220. That is, depending on the training method, the first sub-models 210 and the second sub-models 220 may output only the numerical values corresponding to the factors, or may output both the numerical values and the corresponding factors.

Although a case where the neural network model 200 is operated to measure the body condition 400 for cardiovascular disease, particularly coronary artery disease, will be described as an example below, the types of diseases to which the present disclosure is applied are not limited thereto. Risk factors involved in causing coronary artery disease are widely known. Even a risk factor that has no causal relationship with coronary artery disease has a correlation with a disease, so that it can be used as an important tool to identify the cause of the disease or prevent the disease.

For example, the body characteristic factors that affect coronary artery disease may include age, gender, height, weight, and/or the like, and may further include family history or personal history for coronary artery disease. Furthermore, the pathological characteristic factors that affect coronary artery disease may include the presence/absence of myocardial infarction, the degree of vascular calcification, the stability of blood clots, the intravascular velocity of coronary arteries, the degree of stenosis of coronary arteries, etc. and may further include blood sugar, blood pressure, a cholesterol level, a triglyceride level, obesity, smoking status, etc.

The neural network model 200 according to the present disclosure may be individually trained on and infer how much risk factors related to the causing of coronary artery disease affect coronary artery disease using the electrocardiogram data 300 that can be acquired relatively easily. Furthermore, the neural network model 200 according to the present disclosure may be trained through self-supervised learning, and thus, can independently extract factors related to the causing of coronary artery disease and infer the extent to which each of the factors affects coronary artery disease.

When the first sub-models 210 and the second sub-models 220 are trained through supervised learning, the plurality of first sub-models 210 receive the electrocardiogram data 300 and are individually trained to infer age, gender, height, and weight from the electrocardiogram data 300.

A numerical value related to age, a numerical value related to gender, a numerical value related to height, and a numerical value related to weight may be output from the plurality of first sub models 210. The corresponding numerical number may represent the effect on coronary artery disease. The plurality of second sub-models 220 receive the electrocardiogram data 300, and are individually trained to infer the presence/absence of myocardial infarction, the degree of vascular calcification, the stability of blood clots, the intravascular velocity of coronary arteries, and the degree of stenosis of coronary arteries from the electrocardiogram data 300. A numerical value related to myocardial infarction, a numerical value related to vascular calcification, a numerical value related to the stability of blood clots, a numerical value related to the intravascular velocity of coronary arteries, and a numerical value related to the stenosis of coronary arteries may be individually output from the plurality of second sub-models 220. The corresponding numerical number may represent the effect on coronary artery disease.

The third sub-model 230 may receive a third feature generated based on the numerical values output from the first sub-models 210 and the second sub-models 220. The third feature is a value obtained through the combination performed by the processor 110. The processor 110 may combine the numerical values output from the first sub-models 210 and the second sub-models 220 with weights assigned thereto according to the type of disease. For example, when it is found that there is a high correlation between a specific disease and body characteristic factors, the processor 110 may assign higher weights to the numerical values output from the first sub-models 210. In contrast, when it is found that the influence of body characteristic factors on a specific disease is low and the incidence rate attributable to pathological characteristic factors is high, the processor 110 may assign higher weights to the numerical values output from the second sub-models 220.

The third sub-model 230 may output the body condition 400 for coronary artery disease as a numerical value by using the third feature. The output numerical value may be interpreted as a numerical value indicating the degree of health of coronary arteries, the probability of coronary artery disease, the degree of angina, or the possibility of myocardial infarction. Medical staff may interpret the corresponding value, and may diagnose angina pectoris or make a treatment plan such as the administration of medication or the performance of surgery for a subject whose electrocardiogram data 300 was measured. Alternatively, medical staff may make a plan to prevent disease and provide guidance on the plan.

FIG. 4 is a flowchart showing an inference method for a neural network model according to an embodiment of the present disclosure.

Referring to FIG. 4, the computing device 100 according to an embodiment of the present disclosure may acquire electrocardiogram data in step S110. The computing device 100 may acquire the electrocardiogram data from an electrocardiogram measurement device or acquire it over a network.

The computing device 100 may infer a body condition corresponding to the occurrence or progress of a disease of a subject, whose electrocardiogram data was measured, based on the electrocardiogram data by using a pre-trained neural network model in step S120. The computing device 100 may previously train a neural network model to output a continuous body condition related to a specific disease as a numerical value by using the electrocardiogram data. The neural network model may be one of the neural network models described above with reference to FIG. 2 and 3.

The computing device 100 may train the neural network model based on at least one of a first feature related to biological information representing a body characteristic having a correlation with a disease and a second feature related to pathological information reflecting the degree of progress of the disease therein.

The computing device 100 may train the neural network model through supervised learning or self-supervised learning. More specifically, the computing device 100 may train the neural network model by inputting various factors related to disease and numerical values indicating the correlations between the individual factors and the disease in electrocardiogram data to the neural network model as training data. Alternatively, the computing device 100 may train the neural network model to extract factors related to disease by itself and output numerical values indicating the correlations between the individual factors and the disease by using training data including unlabeled electrocardiogram data.

The pre-trained neural network model may receive electrocardiogram data as input and output a numerical value related to disease. The corresponding numerical value indicates the body condition of a subject whose electrocardiogram data was measured, and is a numerical value related to the disease. The numerical value may indicate whether the disease has occurred, or may indicate the progress of the disease.

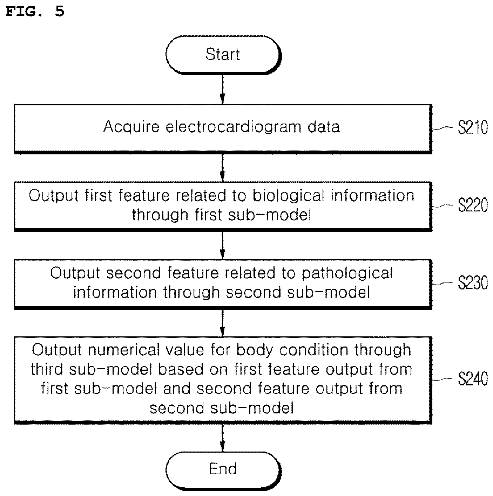

FIG. 5 is a flowchart showing an inference method for sub-models constituting a neural network model according to an embodiment of the present disclosure.

Referring to FIG. 5, the computing device 100 according to an embodiment of the present disclosure may acquire electrocardiogram data in step S210. Since this is similar to step S110 of FIG. 4, a detailed description thereof is omitted.

The computing device 100 may output a first feature related to biological information through the first sub-model in step S220. The first sub-model may be trained to output the first feature based on the electrocardiogram data. The first feature may include at least one factor included in the biological information or a numerical value for the corresponding factor. There may be a plurality of first sub-models, and each of the first sub-models may individually output at least one factor included in the biological information. Accordingly, the number of first sub-models may be configured in accordance with the number of factors.

The computing device 100 may output a second feature related to pathological information through the second sub-model in step S230. The second sub-model may be trained to output the second feature based on the electrocardiogram data. The second feature may include at least one factor included in the pathological information or a numerical value for the corresponding factor. There may be a plurality of second sub-models, and each of the second sub-models may individually output at least one factor included in the pathological information. Accordingly, the number of second sub-models may be configured in accordance with the number of factors.

In this case, each of the first sub-model and the second sub-model may be trained based on self-supervised learning performed using training data including unlabeled samples.

The computing device 100 may output a numerical value for a body condition through the third sub-model based on the first feature output from the first sub-model and the second feature output from the second sub-model in step S240. The third sub-model may be trained to represent a body condition that continuously changes according to the occurrence or progress of a disease as a numerical value based on the first and second features. The computing device 100 may determine weights for the combination of the first feature and the second feature depending on the type of disease. The computing device 100 may generate a third feature by combining the first feature and the second feature based on the determined weights. Accordingly, the third sub-model may receive the third feature as input and output a numerical value indicating the body condition.

Meanwhile, although steps S220 and S230 are shown as being performed sequentially in FIG. 5, steps S220 and S230 may be performed in parallel with each other.

The various embodiments of the present disclosure described above may be combined with one or more additional embodiments, and may be changed within the scope understandable to those skilled in the art in light of the above detailed description. The embodiments of the present disclosure should be understood as illustrative but not restrictive in all respects. For example, individual components described as unitary may be implemented in a distributed manner, and similarly, components described as distributed may also be implemented in a combined form. Accordingly, all changes or modifications derived from the meanings and scopes of the claims of the present disclosure and their equivalents should be construed as being included in the scope of the present disclosure.

## Claims

1. A method of measuring a continuous body condition based on deep learning, the method being performed by a computing device including at least one processor, the method comprising:
acquiring electrocardiogram data; and
inferring a body condition corresponding to an occurrence or progress of a disease in a subject, whose electrocardiogram data was measured, based on the electrocardiogram data by using a pre-trained neural network model;
wherein the neural network model has been trained based on at least one of a first feature related to biological information representing a body characteristic having a correlation with the disease and a second feature related to pathological information reflecting a degree of progress of the disease therein.

2. The method of claim 1, wherein:
the neural network model includes at least one first sub-model trained to output the first feature based on the electrocardiogram data; and
the at least one first sub-model is configured in accordance with a number of factors to individually output numerical values for one or more factors included in the biological information.

3. The method of claim 2, wherein:
the neural network model further includes at least one second sub-model trained to output the second feature based on the electrocardiogram data; and
the at least one second sub-model is configured in accordance with a number of factors to individually output numerical values for one or more factors included in the pathological information.

4. The method of claim 3, wherein the neural network model further includes a third sub-model trained to represent a body condition continuously changing according to the occurrence or progress of the disease in the form of a numerical value based on the first feature, which is an output of the first sub-model, and the second feature, which is an output of the second sub-model.

5. The method of claim 4, wherein the third sub-model receives a third feature generated by combining the first feature and the second feature based on weights determined according to a type of disease, and outputs the numerical value.

6. The method of claim 3, wherein each of the first and second sub-models is trained based on self-supervised learning that is performed using training data including unlabeled samples.

7. The method of claim 1, wherein the disease includes a cardiovascular disease.

8. The method of claim 7, wherein the biological information includes at least one of age, gender, height, and weight as a body characteristic factor related to a coronary artery disease included in the cardiovascular disease.

9. The method of claim 8, wherein the pathological information includes at least one of presence/absence of myocardial infarction, a degree of vascular calcification, stability of blood clots, intravascular velocity of coronary arteries, and a degree of stenosis of coronary arteries as a pathological characteristic factor that reflects a degree of progress of a coronary artery disease included in the cardiovascular disease.

10. A computer program stored in a computer-readable storage medium, the computer program performing operations of measuring a continuous body condition based on deep learning when executed on one or more processors,
wherein the operations include operations of:
acquiring electrocardiogram data; and
inferring a body condition corresponding to an occurrence or progress of a disease in a subject, whose electrocardiogram data was measured, based on the electrocardiogram data by using a pre-trained neural network model; and
wherein the neural network model has been trained based on at least one of a first feature related to biological information representing a body characteristic having a correlation with the disease and a second feature related to pathological information reflecting a degree of progress of the disease therein.

11. A computing device for measuring a continuous body condition based on deep learning, the computing device comprising:
a processor including at least one core;
memory including program codes that are executable on the processor; and
a network unit configured to acquire electrocardiogram data;
wherein the processor infers a body condition corresponding to an occurrence or progress of a disease in a subject, whose electrocardiogram data was measured, based on the electrocardiogram data by using a neural network model trained based on at least one of a first feature related to biological information representing a body characteristic having a correlation with the disease and a second feature related to pathological information reflecting a degree of progress of the disease therein.
